# EUROPEAN PATENT APPLICATION

(11) **EP 4 378 434 A1**
(43) Date of publication of application: **05.06.2024**
(21) Application number: 22210600.7
(22) Date of filing: 30.11.2022
(51) Int. Cl.: A61F 13/00

(54) **PAD FOR TREATING A WOUND COMPRISING AN IMPROVED ULTRASONIC WELD**

(71) Applicant: Paul Hartmann AG, 89522 Heidenheim (DE)
(72) Inventor: MERCKEL, Fabien, 67100 Strasbourg (FR); THIEBAUT, Renaud, 67730 Chatenois (FR)
(74) Representative: Paul Hartmann AG Patents & Licensing

(57) **Abstract**

The present invention relates to a pad (1, 12) for treating a wound, comprising
- a first nonwoven layer (2), said first nonwoven layer (2) intended for facing away from the wound when the pad (1, 12) is positioned over the wound during wound treatment,
- a second nonwoven layer (3, 4), said second nonwoven layer (3, 4) consisting of a different material than the first nonwoven layer (2) and intended for facing towards the wound when the pad (1, 12) is positioned over the wound during wound treatment,
- an ultrasonic weld (5) connecting a border of the first nonwoven layer (2) and a border of the second nonwoven layer (3, 4) with each other such that the first nonwoven layer (2) and the second nonwoven layer (3, 4) form a pocket (6), and
- an absorbent core (7) located within the pocket (6),
characterized in that the first nonwoven layer (2) and the second nonwoven layer (3, 4) comprise plastic fibers consisting of the same material. The pad according to the present invention may be more conformable than prior art absorbent wound pads. A dressing, a production process and a treatment method regarding the pad are also described.

## Description

The present invention relates to a pad for treating a wound comprising a first nonwoven layer, a second nonwoven layer, an ultrasonic weld connecting the nonwoven layers to form a pocket and an absorbent core located within the pocket. In addition, the present invention relates to a dressing comprising the aforementioned pad, a process of producing the aforementioned pad or dressing as well as a use of the aforementioned pad or dressing.

Absorbent wound pads are well known in the prior art. They may, for example, comprise an absorbent core, which is located in a pocket formed by two nonwoven layers. The nonwoven layers may be connected to each other by an ultrasonic weld. Even though such ultrasonic welds are advantageous in many aspects, they may be rigid and thereby decrease the conformability of the wound pad. Moreover, ultrasonic welds may be limited to certain shapes such as a rectangular shape and may be limited in their welding strength.

The task of the present invention was to provide an improved absorbent wound pad. In particular, the task of the present invention was to develop an improved absorbent wound pad with an increased conformability compared to prior art absorbent wound pads. This task was solved by a pad according to claim 1, a dressing according to claim 16, a production process according to claim 17 and a use according to claim 18.

According to the invention, the pad for treating a wound comprises the following components:
- A first nonwoven layer, which is intended for facing away from the wound when the pad is positioned over the wound during wound treatment. The first nonwoven layer may also be considered as a backing layer of the pad as long as no additional layer is permanently attached to the first nonwoven layer forming the outermost structural layer of the pad. Typically, the first nonwoven layer is hydrophobic. Therefore, the first nonwoven layer may be water-repellent and may contribute to retain the absorbed wound exudate within the pad.
- A second nonwoven layer, which consists of a different material than the first nonwoven layer and is intended for facing towards the wound when the pad is positioned over the wound during wound treatment. The second nonwoven layer may also be considered as a wound contact layer of the pad as long as no additional layer is permanently attached to the second nonwoven layer preventing the second nonwoven layer to directly contact the wound. Typically, the second nonwoven layer is hydrophilic to facilitate absorption of wound exudate into the pad. However, the preferred hydrophilic nature of the second nonwoven layer does not rule out that this layer may also comprise hydrophobic fibers. Since the first nonwoven layer and the second nonwoven layer consist of different materials, the first nonwoven layer may be hydrophobic, while the second nonwoven layer may be hydrophilic as described before.
- An ultrasonic weld connecting a border of the first nonwoven layer and a border of the second nonwoven layer with each other such that the first nonwoven layer and the second nonwoven layer form a pocket. Within the present specification, the term "border" may be exchanged with the term "edge" or also "margin".
- An absorbent core located within the pocket.

As already mentioned, the first nonwoven layer and the second nonwoven layer consist of different materials and, consequently, may have different fiber compositions. Nevertheless, according to an essential feature of the present invention the first nonwoven layer and the second nonwoven layer comprise plastic fibers consisting of the same material. In other words, plastic fibers of a certain material are present in the first as well as in the second nonwoven layer. According to the present invention, plastic fibers consist of the "same material" if their chemical composition is identical. Furthermore, according to the present invention the material of the plastic fibers is a fully synthetic material. Therefore, a semisynthetic material such as regenerated cellulose cannot be used for the plastic fibers according to claim 1. Furthermore, the material of the plastic fibers according to the present invention typically has thermoplastic properties. Thus, it is typically a thermoplastic fully synthetic material.

Using plastic fibers of the same material and thus with the same melting point in the nonwoven layers may significantly increase the strength of the ultrasonic weld. The width of the ultrasonic weld may then be reduced, which may result in a less rigid and more conformable pad. Moreover, since less area of the nonwoven layers may be required for the ultrasonic weld, the size of the pocket may increase. The pad may then comprise a larger absorbent core with a higher absorption capacity. The improved ultrasonic weld may also have various shapes including for instance rounded corners, which may further increase the conformability of the pad. Another advantage of the present invention may be a higher production speed of the pad since the ultrasonic welding step may require less time. Preferred embodiments of the present invention may possess further advantages and are described in the following.

It may be advantageous, if the plastic fibers of the first nonwoven layer and the plastic fibers of the second nonwoven layer have the same diameter and/or length. Preferably, the first nonwoven layer and the second nonwoven layer comprise only one type of plastic fibers. In addition, a certain amount of plastic fibers in the first and the second nonwoven layer may be required for ultrasonic welding. Therefore, the first nonwoven layer and the second nonwoven layer may advantageously each comprise at least 30 weight-%, preferably at least 40 weight-%, more preferably at least 50 weight-% and in particular at least 60 weight-% of the plastic fibers. Normally, the first and the second nonwoven layer comprise different amounts of the plastic fibers. For example, the first nonwoven layer may comprise at least 90 weight-% of the plastic fibers, while the second nonwoven layer may comprise between 50 to 70 weight-% of the plastic fibers. In the present specification, weight-% values relate to the total weight of the corresponding layer, unless otherwise stated.

In a preferred embodiment of the present invention the plastic fibers contained in the first nonwoven layer and the second nonwoven layer consist of acrylonitrile butadiene styrene (ABS), polyamide (PA), polylactide (PLA), polymethyl methacrylate (PMMA), polycarbonate (PC), polyethylene terephthalate (PET), polyethylene (PE), polypropylene (PP), polystyrene (PS), polyetheretherketone (PEEK) or polyvinyl chloride (PVC). Preferably, the plastic fibers contained in the first nonwoven layer and the second nonwoven layer consist of polyamide (PA), polyethylene terephthalate (PET), polyethylene (PE), polypropylene (PP) or polyvinyl chloride (PVC). In particular, the plastic fibers contained in the first nonwoven layer and the second nonwoven layer consist of polypropylene. Accordingly, in this particularly preferred embodiment of the present invention polypropylene fibers are present in the first and the second nonwoven layer of the pad.

Preferably, the first nonwoven layer consists of the plastic fibers, in particular polypropylene fibers. The first nonwoven layer may then be hydrophobic. Normally, the first nonwoven layer consists of a single, uniform nonwoven layer. A double-layer construction as described below for the second nonwoven layer is not required for the first nonwoven layer. In general, each layer of the pad consists of a single material layer, unless otherwise stated. Furthermore, the first nonwoven layer may have a basis weight of 20 to 50 g/m², preferably 20 to 30 g/m² and in particular approximately 25 g/m². A rather low basis weight may increase breathability and conformability of the pad.

Usually, the second nonwoven layer further comprises cellulose-based fibers. The second nonwoven layer may then be hydrophilic. Preferably, the cellulose-based fibers are regenerated cellulose fibers, in particular viscose fibers. For instance, the second nonwoven layer may consist of 60 to 70 weight-% of the plastic fibers, preferably polypropylene fibers, and 30 to 40 weight-% cellulose-based fibers, preferably viscose fibers. In particular, the second nonwoven layer may consist of approximately 64 weight-% of the plastic fibers and approximately 36 weight-% cellulose-based fibers.

In a very advantageous and thus preferred embodiment of the present invention, the second nonwoven layer has a double-layer structure and consists of an inner nonwoven layer and an outer nonwoven layer. The inner nonwoven layer forms a part of the inside of the pocket. The outer nonwoven layer forms a part of the outside of the pocket. The inner nonwoven layer may consist of the plastic fibers and cellulose-based fibers and the outer nonwoven layer may consist of the plastic fibers. Accordingly, in the latter embodiment, the inner nonwoven layer is composed of the plastic fibers and cellulose-based fibers, while the outer nonwoven layer is composed only of the plastic fibers. In this configuration, the second nonwoven layer may have atraumatic properties, that is the second nonwoven layer does not adhere to the wound and may be removed from the wound in a gentle as well as painless manner. The inner nonwoven layer and the outer nonwoven layer may be connected to each other by the application of heat.

The inner nonwoven layer may consist of 35 to 45 weight-% of the plastic fibers and 55 to 65 weight-% cellulose-based fibers. In particular, the inner nonwoven layer may consist of approximately 40 weight-% of the plastic fibers and approximately 60 weight-% cellulose-based fibers. The inner and outer nonwoven layer taken together may consist of 60 to 70 weight-% of the plastic fibers and 30 to 40 weight-% cellulose-based fibers, as already mentioned with respect to the (entire) second nonwoven layer.

Moreover, the second nonwoven layer may have a basis weight of 25 to 50 g/m², preferably 40 to 50 g/m² and in particular approximately 45 g/m². If the second nonwoven layer is formed in the double-layer structure mentioned above, the inner nonwoven layer may have a basis weight of 25 to 30 g/m², in particular approximately 27 g/m². The outer nonwoven layer may have a basis weight of 15 to 20 g/m², in particular approximately 18 g/m². Again, the inner and outer nonwoven layer taken together may have a basis weight of 40 to 50 g/m², in particular approximately 45 g/m², as already mentioned with respect to the (entire) second nonwoven layer. A second nonwoven layer with such basis weight values may well absorb and distribute liquids such as wound exudate. Eventually, this may improve the absorption characteristics of the pad.

The liquid distribution capacity (wicking capacity) was also determined by the present inventors. It was found that the second nonwoven layer may have a favorable wicking capacity in machine direction of at least 20 mm after 10 seconds. The wicking capacity of the second nonwoven layer in cross direction can amount to at least 15 mm after 10 seconds. However, in practice the wicking capacity of the second nonwoven layer may also have an upper limit. For example, the wicking capacity of the second nonwoven layer in machine direction can amount to at most 30 mm after 10 seconds. Similarly, the wicking capacity of the second nonwoven layer in cross direction can amount to at most 25 mm after 10 seconds. The wicking capacity in machine direction as well as in cross direction may be determined according to NWSP 010.1.R0 (20). The methodology of the wicking capacity test according to this standard is also described in the present specification.

Typically, the first nonwoven layer and the second nonwoven layer have essentially the same geometrical shape and dimension. For example, both nonwoven layers may be essentially rectangular, preferably essentially rectangular with rounded corners. Suitable dimensions for the nonwoven layers are, for example, 5 × 5 cm, 9 × 9 cm or 14 × 14 cm. Depending on the location and size of the wound to be treated, other shapes and dimensions may be used as well.

The first and second nonwoven layer can be similar in appearance. Therefore, the first nonwoven layer and/or the second nonwoven layer may comprise a visual indicator to indicate which layer is intended to face away and which layer is intended to face towards the wound. The indicator may be a coloring or label. Preferably, the indicator is a coloring, wherein only the first nonwoven layer or the second nonwoven layer is colored. For example, the first nonwoven layer may be colored, for instance in green, while the second nonwoven layer is white and uncolored, respectively. The product insert of the pad may then contain the information for the user that the colored side of the pad is the backing.

In the following, the ultrasonic weld will be described in more detail. Usually, the ultrasonic weld forms an outer circumference of the pad. In addition, the ultrasonic weld is usually formed only between the first and the second nonwoven layer (therefore, apart from the first and second nonwoven layer, no other layers or components are part of the ultrasonic weld and the ultrasonic weld directly connects the first and the second nonwoven layer with each other). Preferably, the ultrasonic weld includes rounded corners. This may improve conformability and softness of the pad. The ultrasonic weld may have a width of 5 mm or less, preferably 2 to 5 mm and in particular 3 to 4 mm. Thus, the ultrasonic weld may be comparatively narrow, leading to the various advantages as explained before.

The inventors also performed tests to determine the welding strength of the ultrasonic weld. The tests showed that the ultrasonic weld may have a welding strength of at least 1.8 N/15 mm, preferably 1.8 to 10 N/15 mm, more preferably 2 to 8 N/15 mm and in particular 4 to 6 N/15 mm. The welding strength may be determined according to the test method described herein, wherein the welding strength relates to the determined average welding strength.

As already mentioned, the pad according to the present invention comprises an absorbent core located within the pocket formed by the first and the second nonwoven layer. The absorbent core preferably comprises a superabsorbent polymer (SAP) and cellulose fibers. In particular, the absorbent core consists of the superabsorbent polymer and the cellulose fibers. For example, the absorbent core may comprise 30 to 60 weight-% superabsorbent polymer and 40 to 70 weight-% cellulose fibers. Preferably, the absorbent core comprises 40 to 50 weight-% superabsorbent polymer and 50 to 60 weight-% cellulose fibers.

Preferably, the superabsorbent polymer is a polyacrylate. Such polyacrylate superabsorbers may bind large amounts of liquid, providing the absorbent core with a high absorption power. Furthermore, it is advantageous if the superabsorbent polymer is present in particulate form. The cellulose fibers of the absorbent core may form a fluff pulp. Preferably, the superabsorbent polymer is distributed and embedded in the fluff pulp.

The pad, in particular the absorbent core of the pad, may have an absorption capacity of at least 50 g/100 cm², preferably of at least 100 g/100 cm² and in particular of at least 140 g/100 cm². These high absorption capacities can be easily achieved by a suitable amount of superabsorbent polymer in the absorbent core, for example in the range of 30 to 60 weight-% as described before. Nevertheless, the absorption capacity of the pad, in particular of the absorbent core, may be limited to 220 g/100 cm², 250 g/100 cm² or 300 g/100 cm². Preferably, the pad, in particular the absorbent core of the pad, has an absorption capacity of 140 g/100 cm² to 220 g/100 cm². The absorption capacity of the pad and absorbent core, respectively, may be determined according to DIN EN 13726-1:2002-06 (Chapter 3.2).

The pad may advantageously comprise further layers. For example, it may be envisaged that the pad further comprises a third nonwoven layer within the pocket, wherein the third nonwoven layer encloses the absorbent core. Consequently, in this embodiment of the present invention a further layer, namely said third nonwoven layer, is located between the absorbent core and the envelope of the pocket formed by the first and the second nonwoven layer. Preferably, the third nonwoven layer comprises cellulose fibers. In particular, the third nonwoven layer consists of cellulose fibers. The third nonwoven layer may serve as a further distribution or wicking layer ensuring that the absorbent core is uniformly wetted with the wound exudate across its entire surface. In this way, the entire absorption capacity of the absorbent core can be utilized.

In addition, the pad may further comprise a perforated, siliconized wound contact layer attached to the second nonwoven layer. The perforations in the wound contact layer allow wound exudate to pass through the wound contact layer into the absorbent pad. Such a wound contact layer may also provide the pad with atraumatic properties and further improve the atraumatic properties of the pad, respectively. In particular, the wound contact layer may comprise a perforated film, wherein a side of the film facing towards the second nonwoven layer of the pad is coated with an acrylic adhesive and an opposite side of the film is coated with a silicone gel. In use, the pad therefore contacts the wound with the atraumatic silicone gel. Preferably, the film consists of polyurethane.

In an embodiment of the present invention the pad comprises only the previously described components or layers and an optional removable release liner. In this embodiment, the pad consists of the first nonwoven layer, the second nonwoven layer, the ultrasonic weld, the absorbent core, optional the third nonwoven layer, optional the wound contact layer and optional the removable release liner. The pad may then be essentially non-adhesive and may be fixed at the tissue site with, for instance, a separate medical adhesive tape or a medical bandage. An advantageous self-adhesive version of the pad is described below in connection with the dressing according to the invention.

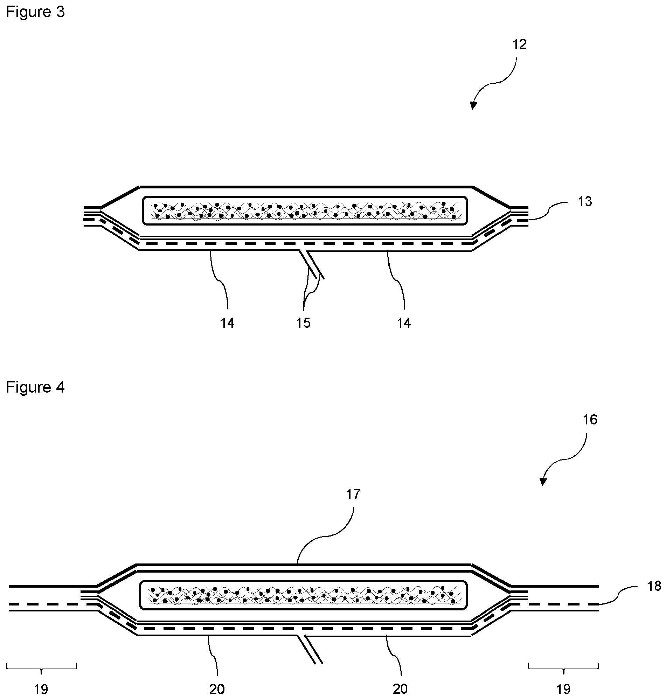

A further subject of the present invention is a dressing for treating a wound. The dressing comprises at least the following three components:
- A pad as previously described.
- A backing layer attached to the first nonwoven layer of the pad. The backing layer is typically water-impermeable and water vapor permeable.
- A perforated, siliconized wound contact layer attached to the second nonwoven layer of the pad. Wound exudate may enter the pad through the perforations.
Importantly, the backing layer and the wound contact layer extend beyond a periphery of the pad and form a border region of the dressing, wherein the backing layer and the wound contact layer are connected to each other in the border region. The border region is intended for contacting intact skin surrounding the wound and for fixing the dressing at the tissue site, providing the dressing with self-adhesive properties. The silicone component of the wound contact layer is mainly responsible for these self-adhesive properties and serves as an atraumatic adhesive. In summary, the proposed dressing may provide a convenient option for the user to easily and quickly fix the pad to the tissue site without the need for additional fixation means (such as the aforementioned adhesive strips or bandages). The dressing usually also comprises the aforementioned removable release liner. The function and a preferred configuration of the release liner is explained in more detail in connection with the figures 3 and 4.

In particular, the wound contact layer of the dressing may comprise a perforated film, wherein a side of the film facing towards the pad is coated with an acrylic adhesive and an opposite side of the film is coated with a silicone gel. Again, the film preferably consists of polyurethane. Even though the silicone gel is generally atraumatic and does not or only slightly adhere to (moist) wound surfaces, it may adhere sufficiently strongly to intact tissue surrounding the wound to safely fix the dressing at the tissue site.

The backing layer of the dressing may comprise a film, wherein a side of the film facing towards the pad is coated with an acrylic adhesive. The film of the backing layer may consist of polyurethane as well. Furthermore, the film may have a thickness of only 15 to 25 µm, in particular approximately 20 µm, for a good breathability. The film can be coated with the acrylic adhesive over the entire surface facing the pad. However, to achieve a higher water vapor permeability and thus breathability of the backing layer it is preferred, that the acrylic adhesive forms a pattern, in particular a grid, on the backing layer. In such a pattern coating, the adhesive may cover 30 to 50 %, preferably 35 to 45 % and in particular approximately 40 % of the backing layer.

Moreover, a process of producing a pad or dressing for treating a wound as previously described is claimed. The process comprises the following steps:
i. Providing a first nonwoven layer, said first nonwoven layer intended for facing away from the wound when the pad or dressing is positioned over the wound during wound treatment.
ii. Providing a second nonwoven layer, said second nonwoven layer consisting of a different material than the first nonwoven layer and intended for facing towards the wound when the pad or dressing is positioned over the wound during wound treatment.
iii. Providing an absorbent core, said absorbent core intended for being enclosed by the first nonwoven layer and the second nonwoven layer.
iv. Covering a first side of the absorbent core with the first nonwoven layer.
v. Covering a second side of the absorbent core with the second nonwoven layer.
vi. Connecting the first nonwoven layer and the second nonwoven layer with each other by ultrasonic welding creating an ultrasonic weld, such that the first nonwoven layer and the second nonwoven layer form a pocket in which the absorbent core is located.
vii. Optional removing excessive parts of the first nonwoven layer and the second nonwoven layer, such that the ultrasonic weld forms a border of the first nonwoven layer and the second nonwoven layer.

The first nonwoven layer and the second nonwoven layer provided in step i. and ii. of the process comprise plastic fibers consisting of the same material.

Another subject of the present invention is directed to the intended medical indication of the pad and dressing, respectively. Accordingly, the pad or dressing as previously described for use in the treatment of a wound is claimed. In other words, the use of the pad or dressing as previously described in the treatment of a wound is claimed. Many different wound types can be successfully treated with the pad and the dressing according to the present invention. However, due to their preferred high absorbency, the pad and the dressing according to the present invention are particularly well suited for the treatment of highly exuding wounds. Therefore, the present pad or dressing is preferably used to treat highly exuding wounds.

Finally, a method of treating a wound is disclosed. The method comprises the following steps:
i. Covering the wound with a pad or dressing as described before.
ii. Fixing the pad or dressing at the wound site. To fix the pad at the wound site, an additional component such as a medical adhesive tape or a medical bandage may be required.

All previously described features of the pad and the dressing are also intended to be disclosed in connection with the production process, the use and the treatment method. Therefore, all previously described features of the pad and the dressing can be included in the production process, the use and the treatment method.

### Figures

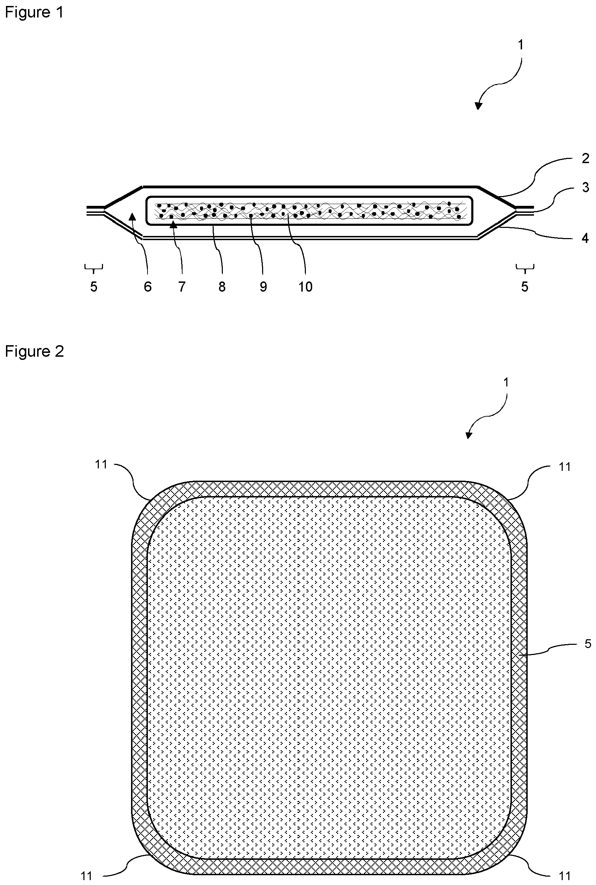

The figures described below are intended to explain and illustrate the invention in more detail by way of example. Similar structural elements may be identified in the figures 1 to 5 with the same reference signs.

**Figure 1** shows a first preferred embodiment of the pad according to the invention in cross section, designated with reference sign **1.** The pad **1** comprises a hydrophobic first nonwoven layer **2** consisting of polypropylene fibers. The first nonwoven layer **2** faces away from the wound when the pad **1** is positioned over the wound during wound treatment. In the embodiment shown in Figure 1, the first nonwoven layer **2** forms the outermost structural layer of the pad **1** when positioned over the wound and therefore constitutes a backing layer of the pad **1.**

Furthermore, pad **1** comprises a hydrophilic second nonwoven layer, which according to the preferred embodiment of Figure 1 consists of two nonwoven layers **3, 4** connected to each other by application of heat. The upper nonwoven layer **3** (also designated inner nonwoven layer in the present specification) consists of a mixture of polypropylene fibers and viscose fibers, preferably in a ratio of approximately 40 weight-% polypropylene fibers and approximately 60 weight-% viscose fibers. In contrast, the lower nonwoven layer **4** (also designated outer nonwoven layer in the present specification) consists similar to the first nonwoven layer **2** solely of polypropylene fibers. Preferably, the upper and lower nonwoven layer **3, 4** together consist of approximately 64 weight-% polypropylene fibers and approximately 36 weight-% viscose fibers. This could also be referred to as the average fiber composition of the second nonwoven layer. The second nonwoven layer **3, 4** faces towards the wound when the pad **1** is positioned over the wound during wound treatment. The second nonwoven layer **3, 4** forms the bottom side of the pad **1** intended for direct wound contact and thus constitutes a wound contact layer of the pad **1.**

The first nonwoven layer **2** may be colored, such as green, while the second nonwoven layer **3, 4** may be white and uncolored, respectively. In this way, the user may easily distinguish the back side of the pad **1** from the wound contact side of the pad **1.**

An ultrasonic weld **5** connects a border of the first nonwoven layer **2** and a border of the second nonwoven layer **3, 4** with each other such that the first nonwoven layer **2** and the second nonwoven layer **3, 4** form a pocket **6.** An absorbent core **7** is located within the pocket 6, which is enclosed by a hydrophilic third nonwoven layer **8** consisting of cellulose fibers. The absorbent core consists of polyacrylate-based superabsorbent polymer particles **9** and cellulose fibers **10** forming a fluff pulp. Preferably, the absorbent core comprises 40 to 50 weight-% superabsorbent polymer particles and 50 to 60 weight-% cellulose fibers. The absorbent core in dry state is very soft and the SAP particles are only loosely embedded in the fluff pulp. However, the enclosure formed by the first and second nonwoven layer **2, 3, 4** hold the absorbent core together. In addition, the third nonwoven layer **8** may support the structural integrity of the absorbent core, even though the main purpose of this layer is to distribute liquid and not to prevent the absorbent core **7** from disintegrating.

During wound treatment, wound exudate enters the pad **1** due to the absorbent and liquid distributing properties of the second nonwoven layer **3, 4** as well as the third nonwoven layer **8.** Finally, the wound exudate is bound in the absorbent core **7.** The hydrophobic nature of the first nonwoven layer **2** may prevent or at least reduce wound exudate from leaking out of the pad **1** when the absorbent core **7** is saturated with wound exudate. The double-layer structure of the second nonwoven layer **3, 4** provides the pad **1** with atraumatic properties without impairing absorbency and liquid distribution. Pad **1** does not contain any adhesive on its outer surfaces. For fixing the pad **1** at the tissue site, a medical tape or bandage can be used.

The inventors found that by incorporating plastic fibers made of the same material, in the case of the embodiment of Figure 1 polypropylene, into the first and the second nonwoven layer the welding strength of the ultrasonic weld **5** can be improved. The ultrasonic weld **5** may then be narrow, preferably only 2 to 5 mm and in particular approximately 3.5 mm, and may comprise rounded corners. Among other things, this may improve conformability of the pad **1.** Incorporating plastic fibers made of the same material into the first and the second nonwoven layer is not self-evident, since the first and the second nonwoven layer serve different functions (hydrophobic backing versus hydrophilic wound contact layer) requiring different fiber compositions in these layers. Moreover, a lot of different plastic fibers are available in the market, many of them being suitable for ultrasonic welding.

**Figure 2** shows the bottom side of the pad **1.** As can be seen in this figure, pad **1** is essentially rectangular with rounded corners **11.** The ultrasonic weld **5** forms an outer circumference of the pad **1.**

**Figure 3** shows a second preferred embodiment of the pad according to the invention in cross section, designated with reference sign **12.** Pad **12** is identical to pad **1** of figure 1, but further comprises a perforated, siliconized wound contact layer **13** attached to the second nonwoven layer **3, 4** of the pad **12.** Preferably, the wound contact layer **13** comprises a perforated polyurethane film as carrier layer. A side of the film facing towards the second nonwoven layer **3, 4** is coated with an acrylic adhesive and an opposite side of the film is coated with a silicone gel (not shown in figure 3). The acrylic adhesive connects the wound contact layer **13** to the second nonwoven layer **3, 4,** while the silicone gel is intended for direct contact with the wound surface. A two-part release liner **14** with removal tabs **15** protects the wound contact layer **13** from contamination during storage. The release liner **14** is peeled off the pad **12** before use of the pad **12** in wound treatment. As already mentioned, the additional wound contact layer **13** may further improve the atraumatic properties of the pad.

**Figure 4** shows a preferred embodiment of the dressing according to the invention in cross section, designated with reference sign **16.** Dressing **16** comprises the pad **1** of figure 1, which is sandwiched between a further backing layer **17** and wound contact layer **18.** The backing layer **17** and the wound contact layer **18** extend beyond a periphery of the pad **1** and form a border region **19** of the dressing **16.** In the border region **19,** the backing layer **17** and the wound contact layer **18** are connected to each other. Preferably, the backing layer **17** comprises a water-impermeable, water vapor permeable polyurethane film, wherein a side of the film facing towards the pad **1** is coated with an acrylic adhesive in a grid pattern. The preferred construction of the wound contact layer **18** is the same as described before in connection with the wound contact layer **13** of figure 3. The connection of the backing layer **17** and the wound contact layer **18** in the border region **19** can then be achieved by the acrylic adhesives of both layers **17, 18.** A removable, two-part release liner **20** is present at the bottom side of the dressing **16** as well. As already pointed out, the dressing **16** may be self-adhesive due to the border region **19.**

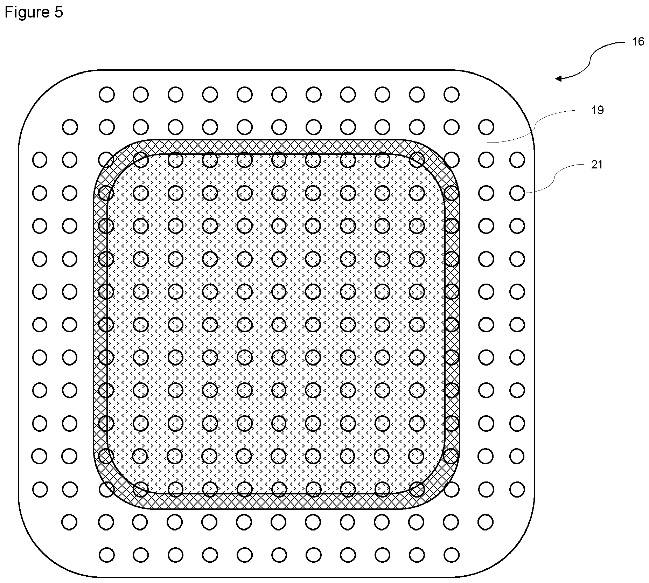

**Figure 5** shows the bottom side of the dressing **16** without the release liner **20.** The pad **1** is located centrally between the backing and wound contact layer **17, 18,** such that the border region **19** may have an essentially equal width. This location of the pad **1** on the backing and wound contact layer, respectively, with the continuous border region **19** is sometimes also called "island design". Since the backing and the wound contact layer **17, 18** are typically transparent, the border region **19** may be transparent as well. This may simplify proper positioning of the dressing **16** at the tissue site. Figure 5 also shows the perforations **21** of the wound contact layer **18,** which are present over the entire surface of the wound contact layer **18** and are arranged in a regular pattern. The central perforations **21** allow wound exudate to pass through the wound contact layer **18** and subsequently be absorbent by the pad **1.** The perforations **21** in the border region **19** may increase the water vapor permeability of the border region **19,** which may eventually reduce skin maceration.

### Wicking capacity test of the second nonwoven layer

The wicking capacity of a preferred embodiment of the second nonwoven layer was determined according to NWSP 010.1 .R0 (20). The tested nonwoven layer consisted of two nonwoven layers connected to each other (aforementioned double-layer structure). One layer consisted of a mixture of 40 weight-% polypropylene fibers and 60 weight-% viscose fibers and had a basis weight of 27 g/m². The other layer consisted solely of polypropylene fibers (that is 100 weight-% polypropylene fibers) and had a basis weight of 18 g/m². Consequently, the tested nonwoven layer in its entirety consisted of 64 weight-% polypropylene fibers and 36 weight-% viscose fibers and had a basis weight of 45 g/m².

Briefly, the following steps are performed for the wicking test:
1 . Cut test specimens 30 mm wide x 250 mm long in both the machine direction and the cross direction.
2. Punch two holes with a diameter of 5 ± 1 mm out of one of the short ends of each test specimen at 5 ± 1 mm from short and long sides.
3. Clamp the test specimen vertically to the horizontal support with the punched holes at the bottom.
4. Place a glass rod through two slots to keep tension on the test specimen and maintain it vertically.
5. Place test specimen neat and parallel to the measuring rod and projecting 15 ± 2 mm below the zero point of the measuring rod.
6. Lower the horizontal support until the zero point of the measuring rod touches the liquid surface. The lower test specimen edge is then 15 ± 2 mm below the surface.
7. At this moment, start stopwatch.
8. Record the height of capillary rise of the liquid after 10, 30 and 60 seconds. If capillary rise is not a uniform straight line, record the highest point.

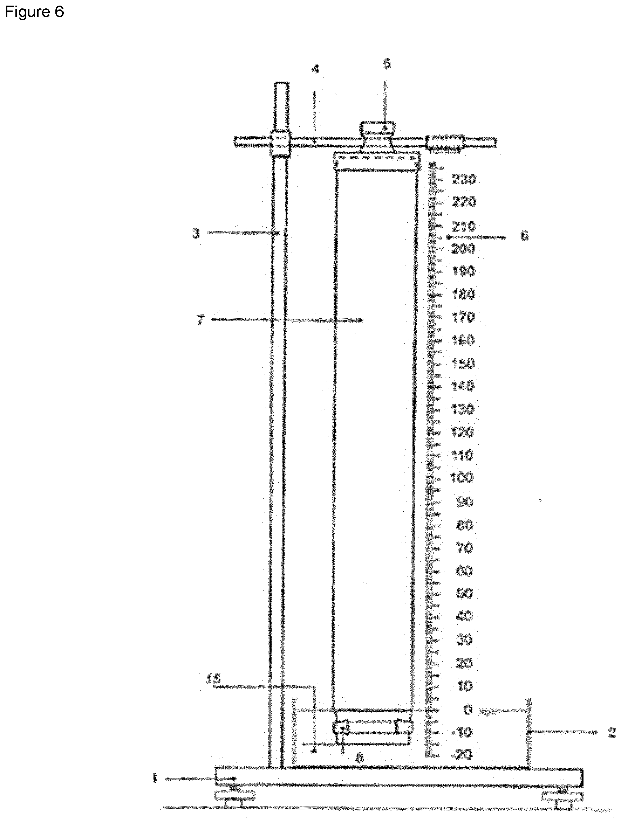

The liquid used in the wicking test is deionized water, equilibrated to room temperature. The measurement apparatus used for the wicking test is shown in **figure 6****.** In contrast to figures 1 to 5, the reference signs 1 to 8 of figure 6 have the following meaning: 1 - base plate; 2 - dish; 3 - vertical support; 4 - horizontal support; 5 - clamps; 6 - measuring rod (scale in mm); 7 - test piece; 8 - glass rod

The test results are summarized in **table 1.** For example, the wicking capacity after 10 seconds was 25 mm in machine direction and 18 mm in cross direction.

**Table 1: Results of the wicking capacity test**

| machine direction | | cross direction | |
|---|---|---|---|
| time [s] | distance [mm] | time [s] | distance [mm] |
| 10 | 25 | 10 | 18 |
| 30 | 35 | 30 | 26 |
| 60 | 44 | 60 | 33 |

### Welding strength test

The welding strength test was performed with the Tensile Tester MTS C42.503E (having a maximum measurable strength/cell strength of 50 N). A pad according to the invention in the embodiment of figure 1 (pad 1) and a reference pad were used for the test. The reference pad was almost identical to the tested pad 1 according to the invention, except for the fact that the second nonwoven layer contained polyamide fibers instead of polypropylene fibers. Therefore, the ultrasonic weld in pad 1 connected a nonwoven layer consisting solely of polypropylene fibers (first nonwoven layer) with a nonwoven layer consisting of polypropylene and viscose fibers (second nonwoven layer), whereas the ultrasonic weld in the reference pad connected a nonwoven layer consisting solely of polypropylene fibers with a nonwoven layer consisting of polyamide and viscose fibers. Thus, in the reference pad the nonwoven layers connected by ultrasonic welding did not contain plastic fibers made of the same material as required by claim 1, but contained plastic fibers made of two different materials, namely polypropylene and polyamide.

Briefly, the following steps are performed for the welding strength test:
1. Stamp 15 × 25 mm samples in the border of the pad.
2. The samples should only consist of the first and second nonwoven layer welded together. Therefore, remove the parts of the third nonwoven layer and the absorbent core contained in each sample.
3. Place the clamp jaws of the tensile tester in a way that the distance between the two jaws is 2 cm.
4. Place each nonwoven in a separate jaw.
5. Start the tensile tester with a speed of 200 mm/min until the two nonwovens are separated. This can happen either after a complete break of the weld or after complete tearing of the nonwovens themselves.
6. Analysis of the measurement data: The welding strength is the average strength over the testing period. It is given in N/15 mm.

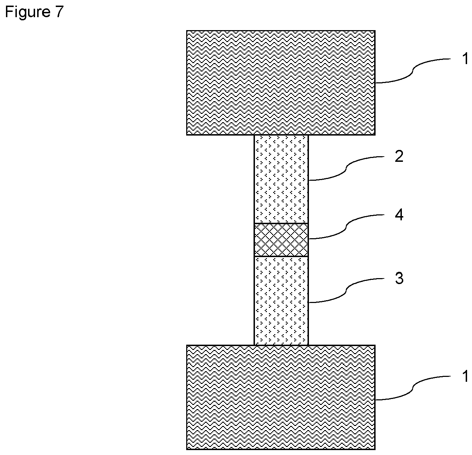

**Figure 7** shows very schematically, how the sample is placed between the jaws. In contrast to the previous figures, the reference signs in figure 7 have the following meaning: 1 -jaw; 2 - first nonwoven layer; 3 - second nonwoven layer; 4 - ultrasonic weld

**Table 2** summarizes the results of the welding strength test. Only for the sake of completeness, table 2 also includes the measured minimum and maximum welding strength values. Surprisingly, it was found that the ultrasonic weld in pad 1 had a significantly higher welding strength than in the reference pad.

**Table 2: Results of the welding strength test**

| welding strength [N/15 mm] | sample from reference pad (not according to the invention) | sample from pad 1 (according to the invention) |
|---|---|---|
| average value | 1.1 | 4.9 |
| minimum value | 0.7 | 2.6 |
| maximum value | 1.1 | 6.9 |

## Claims

1. Pad (1, 12) for treating a wound, comprising
- a first nonwoven layer (2), said first nonwoven layer (2) intended for facing away from the wound when the pad (1, 12) is positioned over the wound during wound treatment,
- a second nonwoven layer (3, 4), said second nonwoven layer (3, 4) consisting of a different material than the first nonwoven layer (2) and intended for facing towards the wound when the pad (1, 12) is positioned over the wound during wound treatment,
- an ultrasonic weld (5) connecting a border of the first nonwoven layer (2) and a border of the second nonwoven layer (3, 4) with each other such that the first nonwoven layer (2) and the second nonwoven layer (3, 4) form a pocket (6), and
- an absorbent core (7) located within the pocket (6),
**characterized in that**
the first nonwoven layer (2) and the second nonwoven layer (3, 4) comprise plastic fibers consisting of the same material.

2. Pad (1, 12) according to claim 1, **characterized in that** the first nonwoven layer (2) and the second nonwoven layer (3, 4) comprise only one type of plastic fibers.

3. Pad (1, 12) according to claim 1 or 2, **characterized in that** the first nonwoven layer (2) and the second nonwoven layer (3, 4) comprise at least 30 weight-% of the plastic fibers, in particular at least 60 weight-% of the plastic fibers.

4. Pad (1, 12) according to anyone of the preceding claims, **characterized in that** the plastic fibers contained in the first nonwoven layer (2) and the second nonwoven layer (3, 4) consist of acrylonitrile butadiene styrene, polyamide, polylactide, polymethyl methacrylate, polycarbonate, polyethylene terephthalate, polyethylene, polypropylene, polystyrene, polyetheretherketone or polyvinyl chloride, in particular polypropylene.

5. Pad (1, 12) according to anyone of the preceding claims, **characterized in that** the first nonwoven layer (2) consists of the plastic fibers.

6. Pad (1, 12) according to anyone of the preceding claims, **characterized in that** the first nonwoven layer (2) has a basis weight of 20 to 50 g/m², preferably 20 to 30 g/m².

7. Pad (1, 12) according to anyone of the preceding claims, **characterized in that** the second nonwoven layer further (3, 4) comprises cellulose-based fibers.

8. Pad (1, 12) according to anyone of the preceding claims, **characterized in that** the second nonwoven layer (3, 4) consists of 60 to 70 weight-% of the plastic fibers and 30 to 40 weight-% cellulose-based fibers.

9. Pad (1, 12) according to anyone of the preceding claims, **characterized in that** the second nonwoven layer (3, 4) consists of an inner nonwoven layer (3) and an outer nonwoven layer (4), wherein the inner nonwoven layer (3) forms a part of the inside of the pocket (6) and wherein the outer nonwoven layer (4) forms a part of the outside of the pocket (6).

10. Pad (1, 12) according to claim 9, **characterized in that** the inner nonwoven layer (3) consists of the plastic fibers and cellulose-based fibers and the outer nonwoven layer (4) consists of the plastic fibers.

11. Pad (1, 12) according to anyone of the preceding claims, **characterized in that** the second nonwoven layer (3, 4) has a basis weight of 25 to 50 g/m², preferably 40 to 50 g/m².

12. Pad (1, 12) according to anyone of the preceding claims, **characterized in that** the second nonwoven layer (3, 4) has a wicking capacity in machine direction of at least 20 mm after 10 seconds and/or a wicking capacity in cross direction of at least 15 mm after 10 seconds.

13. Pad (1, 12) according to anyone of the preceding claims, **characterized in that** the ultrasonic weld (5) includes rounded corners (11).

14. Pad (1, 12) according to anyone of the preceding claims, **characterized in that** the ultrasonic weld (5) has a width of 5 mm or less, preferably 2 to 5 mm and in particular 3 to 4 mm.

15. Pad (1, 12) according to anyone of the preceding claims, **characterized in that** the ultrasonic weld (5) has a welding strength of at least 1.8 N/15 mm, preferably 1.8 to 10 N/15 mm, more preferably 2 to 8 N/15 mm and in particular 4 to 6 N/15 mm.

16. Dressing (16) for treating a wound, comprising
- a pad (1) according to anyone of the preceding claims,
- a backing layer (17) attached to the first nonwoven layer (2) of the pad (1), and
- a perforated, siliconized wound contact layer (18) attached to the second nonwoven layer (3, 4) of the pad (1),
wherein the backing layer (17) and the wound contact layer (18) extend beyond a periphery of the pad (1) and form a border region (19) of the dressing (16), wherein the backing layer (17) and the wound contact layer (18) are connected to each other in the border region (19).

17. Process of producing a pad (1, 12) or dressing (16) for treating a wound according to anyone of the preceding claims, comprising the steps of:
i. providing a first nonwoven layer (2), said first nonwoven layer (2) intended for facing away from the wound when the pad (1, 12) or dressing (16) is positioned over the wound during wound treatment,
ii. providing a second nonwoven layer (3, 4), said second nonwoven layer (3, 4) consisting of a different material than the first nonwoven layer (2) and intended for facing towards the wound when the pad (1, 12) or dressing (16) is positioned over the wound during wound treatment,
iii. providing an absorbent core (7), said absorbent core (7) intended for being enclosed by the first nonwoven layer (2) and the second nonwoven layer (3, 4),
iv. covering a first side of the absorbent core (7) with the first nonwoven layer (2),
v. covering a second side of the absorbent core (7) with the second nonwoven layer (3, 4),
vi. connecting the first nonwoven layer (2) and the second nonwoven layer (3, 4) with each other by ultrasonic welding creating an ultrasonic weld (5), such that the first nonwoven layer (2) and the second nonwoven layer (3, 4) form a pocket (6) in which the absorbent core (7) is located,
vii. optional removing excessive parts of the first nonwoven layer (2) and the second nonwoven layer (3, 4), such that the ultrasonic weld (5) forms a border of the first nonwoven layer (2) and the second nonwoven layer (3, 4),
wherein the first nonwoven layer (2) and the second nonwoven layer (3, 4) comprise plastic fibers consisting of the same material.

18. Pad (1, 12) or dressing (16) according to anyone of the claims 1 to 16 for use in the treatment of a wound.
